# EUROPÄISCHE PATENTANMELDUNG

(11) **EP 2 366 375 A2**
(43) Veröffentlichungstag der Anmeldung: **21.09.2011**
(21) Anmeldenummer: 11157772.2
(22) Anmeldetag: 10.03.2011
(51) Int. Cl.: A61K 8/14, A61K 8/34, A61K 8/41, A61K 8/49, A61Q 19/00

(54) **Zusammensetzung mit einem Sirtuin-Aktivator**

(30) Priorität: 17.03.2010 DE 102010002969
(71) Anmelder: ROVI Cosmetics International GmbH, 36381 Schlüchtern (DE)
(72) Erfinder: Beyer, Monika, 61130, Nidderau-Ostheim (DE); Teichmüller, Dirk, 63589, Linsengericht (DE); Munk, Christian, 36396, Steinau-Marborn (DE); Teichmüller, Sarah, 63589, Linsengericht (DE)
(74) Vertreter: WSL Patentanwälte

(57) **Zusammenfassung**

Die vorliegende Erfindung betrifft eine Zusammensetzung mit wenigstens einem Sirtuin-Aktivator und mit einem Trägersystem für den wenigstens einen Sirtuin-Aktivator, wobei das Trägersystem Lipidvesikel mit einer oder mehreren Lipidmembran(en) umfasst. Um eine verbesserte Wirkung von Sirtuin-Aktivatoren in lebenden Hautzellen zur Verfügung zu stellen, wird erfindungsgemäß vorgeschlagen, dass der wenigstens eine Sirtuin-Aktivator in den Lipidvesikeln enthalten ist und die Lipidvesikel eine positive Oberflächenladung aufweisen, wobei die positive Oberflächenladung dadurch bewirkt wird, dass die Lipidvesikel in der/den Lipidmembran/en zusätzlich zu den Lipiden, aus denen die Vesikel aufgebaut sind, positiv geladene Moleküle als Ladungsgeber aufweisen, wobei diese Ladungsgeber unter quartären Ammoniumverbindungen ausgewählt sind.

## Beschreibung

Die vorliegende Erfindung betrifft Zusammensetzungen mit wenigstens einem Sirtuin-Aktivator und mit einem Trägersystem für den wenigstens einen Sirtuin-Aktivator, wobei das Trägersystem Lipidvesikel mit einer oder mehreren Lipidmembran(en) umfasst, sowie Sirtuin-Aktivatoren enthaltende Wirkstoffzusammensetzung, die Verwendung solcher Zusammensetzungen und Wirkstoffzusammensetzungen und solche Zusammensetzungen bzw. Wirkstoffzusammensetzungen enthaltende kosmetische und/oder pharmazeutische Formulierungen.

Die Familie der Sirtuine, die auch SIRT-Enzyme (Silent Information Regulator) genannt werden, gehört zu den NAD(+) abhängigen Histon-Deacetylasen. Sie regulieren wichtige biologische Prozesse, wie die Zellalterung bzw. Zelllanglebigkeit, Apoptose, Zelldifferenzierung und den Zellstoffwechsel im Allgemeinen. Es wurde gezeigt, dass eine erhöhte Aktivität von Sirtuinen, die z. B. durch Nahrungsmangel ausgelöst wird, eine lebensverlängernde Wirkung auf Zellen ausübt und den Alterungsprozess sowie stressinduzierten programmierten Zelltod verzögert.

Es wurde weiter festgestellt, dass derartige Einflüsse auf die Lebensdauer von Zellen neben der Nahrungsmittelverknappung auch durch bestimmte Wirkstoffe, die Sirtuin-Aktivatoren, ausgeübt werden. In der Literatur werden zahlreiche derartige Sirtuin-Aktivatoren beschrieben, u.a. Resveratrol und Derivate davon, die die Aktivität von Sirtuinen erhöhen.

Eine Schwierigkeit bei der Verwendung derartiger Sirtuin-Aktivatoren ergibt sich dadurch, dass diese zu den Zielenzymen, den Sirtuinen gelangen müssen und dort eine möglichst gute Aktivität, d.h. möglichst starke Steigerung der Aktivität der Sirtuine, entfalten sollen. Insbesondere bei der Verminderung von Hautalterung ist es dabei notwendig, dass die Sirtuin-Aktivatoren durch die verhornten Schichten der Oberhaut gelangen.

Ein weiteres Problem bei der Steigerung der Langlebigkeit von Hautzellen besteht darin, die in den Hautzellen vorhandenen spezifischen Sirtuine zu aktivieren.

Der vorliegenden Erfindung liegt daher die Aufgabe zugrunde, eine verbesserte Wirkung von Sirtuin-Aktivatoren in lebenden Hautzellen zur Verfügung zu stellen.

Diese Aufgabe wird erfindungsgemäß gelöst durch eine Zusammensetzung der eingangs genannten Art, bei der der wenigstens eine Sirtuin-Aktivator in den Lipidvesikeln enthalten ist und die Lipidvesikel eine positive Oberflächenladung aufweisen, wobei die positive Oberflächenladung dadurch bewirkt wird, dass die Lipidvesikel in der/den Lipidmembran/en zusätzlich zu den Lipiden, aus denen die Vesikel aufgebaut sind, positiv geladene Moleküle als Ladungsgeber aufweisen, wobei diese Ladungsgeber unter quartären Ammoniumverbindungen ausgewählt sind.

Unter quartären Ammoniumverbindungen versteht man organische Verbindungen, in denen eine quartäre Aminogruppe enthalten ist, wobei in der quartären Aminogruppe vier Valenzen des Stickstoffatoms gebunden sind und das Stickstoffatom eine positive Ladung aufweist.

Die Sirtuin-Aktivatoren befinden sich entweder im Vesikelinnenraum und/oder in der Vesikelmembran. Bei bestimmten Ausführungsformen befinden sich ein oder mehrere Sirtuin-Aktivatoren in der Vesikelmembran und ein oder mehrere andere Sirtuin-Aktivatoren im Vesikelinnenraum.

Es hat sich gezeigt, dass durch die Verwendung eines Trägersystems mit Lipidvesikeln, in die die Sirtuin-Aktivatoren eingebracht sind, wobei die Lipidvesikel quartäre Ammoniumverbindungen als Ladungsträger aufweisen, die Verfügbarkeit und damit die Wirksamkeit von Sirtuin-Aktivatoren in Zellen, insbesondere Hautzellen, deutlich erhöht wird.

Die verbesserte Verfügbarkeit des applizierten Wirkstoffs basiert u. a. auf der positiven Ladung der Vesikeloberfläche. Die positive Ladung der Vesikeloberfläche führt zu einer verbesserten Anhaftung der Vesikel an der Oberfläche von Zellen, insbesondere lebenden Hautzellen. Dies bedingt, dass der in den Vesikeln enthaltene Sirtuin-Aktivator gezielt in lebenden Hautzellen zur Verfügung gestellt wird.

Hierdurch wird die Menge an Sirtuin-Aktivator(en), die in den Hautzellen bereitgestellt wird, signifikant erhöht und damit auch deren Wirksamkeit verbessert, wobei diese Feststellungen in Bezug auf die vorliegende Erfindung jedoch nicht bindend sein sollen und den Umfang der Erfindung auch nicht beschränken sollen.

Besonders bevorzugt sind die quartären Ammoniumverbindungen unter Alkyl-Trimoniumsalz der folgenden Formel ausgewählt: 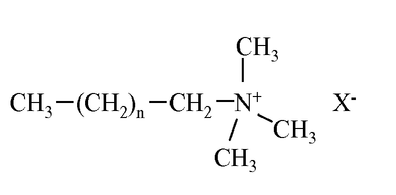 wobei
n eine Zahl von 18 bis 28 und
X- ein anorganisches oder organisches Anion ist.

Derartige Verbindungen haben sich als besonders geeignet für den Transport von Sirtuin-Aktivatoren in Hautzellen erwiesen. Die durch diese Ladungsträger positiv geladenen Vesikel ermöglichen eine besonders gute Penetration der Sirtuin-Aktivatoren in Hautzellen und dadurch eine gezielte Bereitstellung der Wirkstoffe.

Vorzugsweise beträgt der Anteil an Sirtuin-Aktivator(en) bei der vorliegenden Erfindung bezogen auf die gesamte Zusammensetzung 0,0001 bis 20 Gew.-%, bevorzugt, 0,0005 bis 15 Gew.-%, besonders bevorzugt 0,001 bis 10 Gew.-%.

Vorzugsweise ist der wenigstens eine Sirtuin-Aktivator ausgewählt aus der Gruppe, bestehend aus Flavonen, Stilbenen, Flavononen, Isoflavonen, Catechinen, Chalkonen, Tanninen, Anthocyaniden und deren Derivaten. Bevorzugte Sirtuin-Aktivatoren sind Quercitin, Piceatannol, Resveratrol, Isonicotinamid (auch Isoniacinamid genannt), Butein, Isoliquiritgenin, Fisetin, Luteolin und 3,6,3',4'-Tetrahdroxyflavon. Zusätzlich umfassen die Sirtuin-Aktivatoren in bestimmten Ausführungsformen cis- oder trans-Isomere der Substanzen oder eine Kombination aus beiden Isomeren. Besonders bevorzugte Sirtuin-Aktivatoren sind ausgewählt unter Quercitin, Piceatannol, Resveratrol und Isonicotinamid.

In einer bevorzugten Ausführungsform der Erfindung sind wenigstens zwei Sirtuin-Aktivatoren von der Zusammensetzung umfasst. Dabei bedeutet "wenigstens zwei", dass genau zwei, drei, vier, fünf oder mehr Sirtuin-Aktivatoren in der Zusammensetzung enthalten sein können. Durch das Vorhandensein von mehreren Sirtuin-Aktivatoren wird ein Zusammenwirken der Aktivatoren ermöglicht. Bevorzugt sind die wenigstens zwei Sirtuin-Aktivatoren ausgewählt aus der Gruppe, bestehend aus Quercitin, Piceatannol, Resveratrol und Isonicotinamid.

Besonders bevorzugt sind Zusammensetzung, die die Sirtuin-Aktivatoren Resveratrol und Isonicotinamid umfassen. Diese Kombination von Sirtuin-Aktivatoren hat sich als besonders geeignet für die Aktivierung von Sirtuinen erwiesen. Die Sirtuin-Aktivatoren weisen dabei eine synergistische Wirkung im Vergleich zu anderen Wirkstoffkombinationen auf. Es wird angenommen, dass diese auf den unterschiedlichen Angriffspunkten der beiden Wirkstoffe, entweder an einem bestimmten Sirtuin und/oder an unterschiedlichen Sirtuinen beruht. Die Erfindung soll jedoch nicht an diese Theorie gebunden sein und der Umfang der Erfindung soll durch diese Theorie auch nicht beschränkt werden.

Bevorzugt beträgt der Anteil der Sirtuin-Aktivatoren bei einer Resveratrol und Isonicotinamid enthaltenden Zusammensetzung bezogen auf die gesamte Zusammensetzung 0,001 bis 1 Gew.-% Resveratrol und 0,1 bis 10 Gew.-% Isonicotinamid. In diesen Mengenbereichen wird eine besonders gute Aktivierung beobachtet.

Vorzugsweise ist n in der oben angegebenen Alkyl-Trimoniumsalzformel gleich 22. Inbesondere bevorzugt ist das Alkyl-Trimoniumsalz Behentrimoniumchlorid. Die durch das Behentrimoniumchlorid positivierten Lipidvesikel ermöglichen einen zielgerichteten Transport der Sirtuin-Aktivatoren in die Zelle.

Vorzugsweise ist X- ein Halogenid-lon oder das Anion einer organischen Säure, die ausgewählt ist unter einer kosmetisch oder pharmazeutisch verträglichen Carbonsäure oder Sulfonsäure. Besonders bevorzugt ist X⁻ Bromid, Chlorid, Fluorid, lodid, Saccharinat, Tosylat oder Methosulfat.

Vorzugweise werden die positiv geladenen quartären Ammoniumverbindungen in einer Menge von 0,01 bis 10 Gew.-%, bevorzugt 0,01 bis 2,0 Gew.-%, bezogen auf die gesamte Zusammensetzung eingesetzt.

Vorzugsweise haben die Lipidvesikel ein Zetapotential im Bereich von 1 bis 150 mV. Der Begriff "Zetapotential" beschreibt das elektrische Potential einer Abscherschicht eines bewegten Partikels in einer Suspension. Die Messung des Zetapotentials kann dadurch erfolgen, dass man Partikel durch ein angelegtes elektrisches Feld bewegt. Aus der resultierenden Geschwindigkeit der Partikel lässt sich dann das Zetapotential berechnen.

Bei bestimmten Ausführungsformen haben die Lipidvesikel ein Zetapotential im Bereich von 30 bis 100 mV auf. Bei bevorzugten Lipidvesikeln beträgt das Zetapotential 40 bis 60 mV.

Die Teilchengröße der erfindungsgemäßen Lipidvesikel beträgt vorzugsweise von 10 bis 1000 nm, 100 bis 400 nm, mehr bevorzugt 100 bis 350 nm, am meisten bevorzugt 100 bis 250 nm.

Die erfindungsgemäße Zusammensetzung kann Vesikel mit einer Lipidmembran (Nanosomen), zwei Lipidmembranen (Liposomen) oder mehreren Lipidmembranen umfassen.

Die Lipide sind bevorzugt unter Ceramiden, Phospholipiden, Glycosphingolipiden und/oder Di-Acylglycosiden ausgewählt. Dazu gehören auch Sphingomyeline, Galactocerebroside und Glucocerebroside, Dihexoside, Tri- und Tetrahexoside sowie Ganglioside. Die Phospholipide, die bei der Zusammensetzung für die Bildung der Vesikel verwendet werden können, können unter allen kosmetisch oder pharmazeutisch verträglichen Phospholipiden ausgewählt werden, die in der Lage sind, in wässrigem Milieu Vesikel (Nanosomen oder Liposomen) zu bilden. Bevorzugte Phospholipide sind Lecithin, Phosphatidylcholin, Phosphatidylethanolamin und Phosphatidylserin. Besonders bevorzugt ist Lecithin mit einem hohen Anteil an Phosphatidylcholin. Bei speziellen Ausführungsformen können auch Gemische der oben genannten Lipide eingesetzt werden.

In einer Ausführungsform beträgt der Anteil des Lipids /der Lipide bezogen auf die gesamte Zusammensetzung vorzugsweise 1 bis 35 Gew.-%, bevorzugt 3 bis 20 Gew.-%, besonders bevorzugt 5 bis 11 Gew.-%.

Erfindungsgemäß sind auch Wirkstoffzusammensetzungen, die wenigstens zwei Sirtuin-Aktivatoren umfassen, wobei die wenigstens zwei Sirtuin-Aktivatoren ausgewählt sind aus der Gruppe, bestehend aus Quercitin, Piceatannol, Resveratrol und Isonicotinamid. Bevorzugt umfassen die zwei Sirtuin-Aktivatoren die Verbindungen Resveratrol und Isonicotinamid. Gerade bei der Steigerung der Langlebigkeit von Hautzellen und der damit verbundenen Reduzierung der Alterung der Haut hat sich die Kombination von Resveratrol und Isonicotinamid als besonders geeignet erwiesen. Diese Sirtuin-Aktivatoren weisen dabei eine synergistische Wirkung im Vergleich zu anderen Wirkstoffkombinationen auf. Es wird angenommen, dass diese auf den unterschiedlichen Angriffspunkten der beiden Wirkstoffe, entweder an einem bestimmten Sirtuin und/oder an unterschiedlichen Sirtuinen beruht. Die Erfindung soll jedoch nicht an diese Theorie gebunden sein und der Umfang der Erfindung soll durch diese Theorie auch nicht beschränkt werden.

In einer bevorzugten Ausführungsform beträgt das Verhältnis von Resveratrol zu Isonicotinamid in der Wirkstoffzusammensetzung von 0,001 zu 10 bis 10 zu 1.

In einer Ausführungsform weist die Wirkstoffzusammensetzung ein für Sirtuin-Aktivatoren geeignetes Trägersystem auf, wobei das Trägersystem unter neutralen und positiv geladenen Lipidvesikeln ausgewählt ist. Bevorzugt sind positiv geladenen Lipidvesikel. Durch das Trägersystem der Wirkstoffzusammensetzung wird insbesondere mit positiv geladenen Lipidvesikeln die in den Hautzellen bereitgestellte Menge an Sirtuin-Aktivatoren signifikant erhöht, was letztlich zu einer gesteigerten Wirksamkeit der Wirkstoffzusammensetzung führt.

Die positive Ladung der Vesikeloberfläche kann auf verschiedene Weisen erreicht werden. Bei einer Ausführungsform der Wirkstoffzusammensetzung verleihen die oder wenigstens ein Teil der die Lipidmembran bildenden Lipide der Vesikeloberfläche eine positive Ladung. In einer bevorzugten Ausführungsform der Wirkstoffzusammensetzung umfassen die Lipidvesikel zusätzlich zu den Lipiden, aus denen die Vesikel aufgebaut sind, positiv geladene Moleküle als Ladungsgeber.

In einer bestimmten Ausführungsform entsprechen die positiv geladenen Moleküle in den Lipidvesikeln der Wirkstoffzusammensetzung den quartären Ammoniumverbindungen der erfindungsgemäßen Zusammensetzung.

In bestimmten Ausführungsformen umfasst die Zusammensetzung oder Wirkstoffzusammensetzung zusätzlich einen oder mehrere weitere Wirkstoffe. Dabei gehören die weiteren Wirkstoffe nicht zu der Gruppe der Sirtuin-Aktivatoren und umfassen bevorzugt Vitamine, Glucoside, Antioxidantien und Anti-Ageing-Wirkstoffe. Derartige weitere Wirkstoffe erhöhen die Überlebenfähigkeit der Zellen, verlangsamen die Zellalterung und/oder schützen die Zellen vor Schädigungen. Bevorzugte weitere Wirkstoffe sind Tocopherole (Vitamin E) bzw. Glyceryl-Glucoside. In bestimmten Ausführungsformen ist ein Tocopherol in einer Menge von 0,1 bis 3 Gew.-% und/oder ein Glyceryl-Glucosid in einer Menge von 0,1 bis 10 Gew.-% bezogen auf die gesamte Zusammensetzung enthalten.

Erfindungsgemäß wird die Zusammensetzung bzw. die Wirkstoffzusammensetzung zur Herstellung einer Formulierung verwendet, die für Behandlung von Hautalterung und mit Hautalterung assoziierten Symptomen und zur Steigerung der Langlebigkeit von Hautzellen geeignet ist. Zu den Formulierungen zählen kosmetische und/oder pharmazeutische Formulierungen, wobei pharmazeutische Formulierungen solche sind, die unter das Arzneimittelrecht fallen. Dabei können der Zusammensetzung, die positiv geladene Lipidvesikel und wenigstens einen Sirtuin-Aktivator umfasst, weitere Wirkstoffe hinzugefügt werden, die entweder in den Lipidvesikeln oder außerhalb der Lipidvesikel und in der übrigen Substanz bzw. Trägermatrix der Formulierung enthalten sind. Das gleiche trifft für die Wirkstoffzusammensetzung zu.

Die Formulierungen können alle Hilfs- und Zusatzstoffe, die üblicherweise bei kosmetischen oder pharmazeutischen Präparaten Anwendung finden, enthalten. Insbesondere fallen unter den Begriff "Hilfsstoff" im Zusammenhang mit der vorliegenden Erfindung solche Zusatzstoffe, die auf die physikalischen Eigenschaften der Wirkstoffe und/oder Vesikel und deren Stabilität einwirken und/oder der Konservierung der Zusammensetzung oder Wirkstoffzusammensetzung dienen. Beispiele für solche Hilfsstoffe sind Öle, Alkohole, Polyole, Gelbildner, Puffer, Konservierungsmittel, Bakterizide und Keimhemmer, Komplexbildner, Verdicker oder Konsistenzgeber.

Bevorzugt werden die erfindungsgemäßen Zusammensetzung, Wirkstoffzusammensetzungen bzw. Formulierungen für kosmetische und/oder pharmazeutische Formulierungen verwendet, die zur topischen Applikation geeignet sind. Die erfindungsgemäße Zusammensetzung und Wirkstoffzusammensetzung kann in allen für die topische Applikation geeigneten Formulierungen vorliegen, beispielsweise in Form eines Gels, einer Creme, einer Salbe, eines Sprays oder einer Lotion. Dazu kann die erfindungsgemäße Zusammensetzung oder die erfindungsgemäße Wirkstoffzusammensetzung in eine Trägermatrix eingearbeitet werden. Bei der Trägermatrix kann es sich um Gelformulierungen, Cremeformulierungen, Lotionen, Maskenanwendungen etc. handeln.

Für Anwendungen im Hautbereich wird die erfindungsgemäße Zusammensetzung oder die erfindungsgemäße Wirkstoffzusammensetzung vorzugsweise in einer Lotion, einer Creme, einer Salbe, einem Gel, einem wässrigen Fluid, einem Gesichtswasser oder einer Maske angewendet.

Selbstverständlich handelt es sich bei allen Komponenten der erfindungsgemäßen Zusammensetzungen, Wirkstoffzusammensetzungen und Formulierungen um pharmazeutisch, kosmetisch oder dermatologisch verträgliche Stoffe. Ein Stoff ist im Sinne dieser Erfindung pharmazeutisch, kosmetisch oder dermatologisch verträglich, wenn er nicht toxisch ist und bei der Mehrzahl der potentiellen Anwender topisch anwendbar ist, ohne dass es bei dem Anwender spontan oder nach einer Weile zu einer ungewünschten physiologischen Reaktion, wie z.B. einer Rötung oder der Ausbildung eines Juckreizes kommt.

Für Zwecke der ursprünglichen Offenbarung wird darauf hingewiesen, dass sämtliche Merkmale, wie sie sich aus der vorliegenden Beschreibung und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder chemische, physikalisch-chemische, kosmetische, pharmakologische oder dermatologische Gegebenheiten derartige Kombinationen unmöglich oder sinnlos machen. Auf die umfassende, explizite Darstellung sämtlicher denkbaren Merkmalskombinationen wird hier nur der Kürze und der Lesbarkeit der Beschreibung wegen verzichtet.

Des Weiteren wird darauf hingewiesen, dass es für den Fachmann selbstverständlich ist, dass das nachfolgende Beispiel lediglich dazu dient, eine mögliche Ausführungsformen der vorliegenden Erfindung beispielhaft anzugeben. Der Fachmann wird daher ohne weiteres verstehen, dass darüber hinaus auch alle anderen Ausführungsformen, die die in den Ansprüchen genannten erfindungsgemäßen Merkmale oder Merkmalskombinationen aufweisen, innerhalb des Schutzumfangs der Erfindung liegen. Auf die umfassende, explizite Darstellung sämtlicher denkbarer Ausführungsformen innerhalb der beanspruchten Erfindung wird hier nur der Kürze und der Lesbarkeit der Beschreibung wegen verzichtet.

### Beispiel

Ein erfindungsgemäßes Beispiel betrifft eine pharmazeutische Formulierung mit der folgenden Zusammensetzung:
Phase A:
   12, 00 Gew.-% Ethanol
   3,00 Gew.-% Behentrimoniumchlorid
   7,00 Gew.-% Lecithin
   0,60 Gew.-% Resveratrol
   1,00 Gew.-% Tocopherol
Phase B:
   0,50 Gew.-% Isoniacinamid
   74,35 Gew.-% Wasser
   1,00 Gew.-% Glyceryl-Glucosid
Phase C:
   0,50 Gew.-% Kaliumphosphat
   0,05 Gew.-% Natriumhydroxid

Um die Vesikel gemäß dem Beispiel herzustellen, wurden die Bestandteile der Phase A in Ethanol gelöst und die Bestandteile der Phase B in Wasser gelöst. Anschließend wurde Phase B zu Phase A gegeben und mit Hochdruck homogenisiert. Zur Fertigstellung der Zusammensetzung wurde Phase C hinzugegeben.

Die durch das Lecithin gebildeten Vesikel weisen eine Teilchengröße von 100 bis 300 nm auf. Das Resveratrol ist in der Vesikelmembran enthalten, während das Isoniacinamid im Vesikelinnenraum vorliegt und von der Lipidschicht umkapselt ist.

## Patentansprüche

1. Zusammensetzung mit wenigstens einem Sirtuin-Aktivator und mit einem Trägersystem für den wenigstens einen Sirtuin-Aktivator, wobei das Trägersystem Lipidvesikel mit einer oder mehreren Lipidmembran(en) umfasst, **dadurch gekennzeichnet, dass** der wenigstens eine Sirtuin-Aktivator in den Lipidvesikeln enthalten ist und die Lipidvesikel eine positive Oberflächenladung aufweisen, wobei die positive Oberflächenladung dadurch bewirkt wird, dass die Lipidvesikel in der/den Lipidmembran/en zusätzlich zu den Lipiden, aus denen die Vesikel aufgebaut sind, positiv geladene Moleküle als Ladungsgeber aufweisen, wobei diese Ladungsgeber unter quartären Ammoniumverbindungen ausgewählt sind.

2. Zusammensetzung gemäß Anspruch 1, **dadurch gekennzeichnet, dass** die Ladungsgeber aus Alkyl-Trimoniumsalzen der Formel 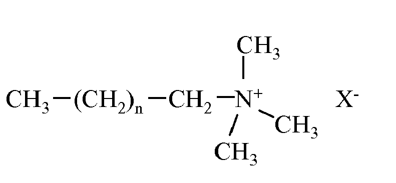 ausgewählt sind, wobei
n eine Zahl von 18 bis 28 und
X- ein anorganisches oder organisches Anion ist.

3. Zusammensetzung gemäß einem der Ansprüche 1 oder 2, **dadurch gekennzeichnet, dass** der Anteil des wenigstens einen Sirtuin-Aktivators bezogen auf die gesamte Zusammensetzung 0,0001 bis 20 Gew.-% beträgt.

4. Zusammensetzung gemäß einem der vorangehenden Ansprüche, **dadurch gekennzeichnet, dass** der wenigsten eine Sirtuin-Aktivator ausgewählt ist unter Flavonen, Stilbenen, Flavononen, Isoflavonen, Catechinen, Chalkonen, Tanninen, Anthocyaniden und deren Derivaten.

5. Zusammensetzung gemäß einem der vorangehenden Ansprüche, **dadurch gekennzeichnet, dass** die Zusammensetzung wenigstens zwei Sirtuin-Aktivatoren umfasst, wobei die wenigstens zwei Sirtuin-Aktivatoren ausgewählt sind aus der Gruppe, bestehend aus Quercitin, Piceatannol, Resveratrol und Isonicotinamid.

6. Zusammensetzung gemäß Anspruch 5, **dadurch gekennzeichnet, dass** die wenigstens zwei Sirtuin-Aktivatoren Resveratrol und Isonicotinamid umfassen.

7. Zusammensetzung gemäß einem der vorangehenden Ansprüche, **dadurch gekennzeichnet, dass** der Anteil der Ladungsgeber bezogen auf die gesamte Zusammensetzung 0,01 bis 10 Gew.-% beträgt.

8. Zusammensetzung gemäß Anspruch 2, **dadurch gekennzeichnet, dass** X- Bromid, Chlorid, Fluorid, lodid, Saccharinat, Tosylat oder Methosulfat ist.

9. Zusammensetzung gemäß einem der vorangehenden Ansprüche, **dadurch gekennzeichnet, dass** das Zetapotential der Lipidvesikel von 1 bis 150 mV beträgt.

10. Zusammensetzung gemäß einem der vorangehenden Ansprüche, **dadurch gekennzeichnet, dass** die Lipidvesikel eine Teilchengröße von 10 bis 1000 nm haben.

11. Wirkstoffzusammensetzung, die wenigstens zwei Sirtuin-Aktivatoren umfasst, wobei die wenigstens zwei Sirtuin-Aktivatoren Resveratrol und Isonicotinamid umfassen.

12. Wirkstoffzusammensetzung gemäß Anspruch 11, **dadurch gekennzeichnet, dass** sie ein für Sirtuin-Aktivatoren geeignetes Trägersystem aufweist, das ausgewählt ist unter neutralen Lipidvesikeln und positiv geladenen Lipidvesikeln.

13. Zusammensetzung gemäß einem der Ansprüche 1 bis 10 oder Wirkstoffzusammensetzung gemäß einem der Ansprüche 11 oder 12, **dadurch gekennzeichnet, dass** sie zusätzlich einen oder mehrere weitere Wirkstoffe umfasst.

14. Verwendung einer Zusammensetzung gemäß einem der Ansprüche 1 bis 10 oder 13 oder einer Wirkstoffzusammensetzung gemäß einem der Ansprüche 11 bis 13 für die Herstellung einer Formulierung für die Behandlung von Hautalterung und mit Hautalterung assoziierten Symptomen und zur Steigerung der Langlebigkeit von Hautzellen.

15. Kosmetische und/oder pharmazeutische Formulierung für die topische Applikation, **dadurch gekennzeichnet, dass** sie eine Zusammensetzung gemäß einem der Ansprüche 1 bis 10 oder 13 und/oder eine Wirkstoffzusammensetzung gemäß einem der Ansprüche 11 bis 13 enthält.
